# EUROPEAN PATENT SPECIFICATION

(11) **EP 1 908 440 B1**
(45) Date of publication and mention of the grant of the patent: **23.12.2009**
(21) Application number: 07019554.0
(22) Date of filing: 05.10.2007
(51) Int. Cl.: A61F 7/03

(54) **Expendable heating sheet, method of manufacturing the same, and heating sheet bag for the same**
Einmal-Heizdecke, Verfahren zu ihrer Herstellung und Heizdeckentasche dafür
Plaque de chauffage extensible, son procédé de fabrication, et emballage de plaque de chauffage pour celle-ci

(30) Priority: 06.10.2006 JP 2006275351
(43) Date of publication of application: 09.04.2008
(73) Proprietor: JAPAN PIONICS CO., LTD., Minato-ku, Tokyo (JP)
(72) Inventor: Ajiri, Asao, Hiratsuka-shi Kanagawa (JP); Matsumoto, Yoshiki, Hiratsuka-shi Kanagawa (JP)
(74) Representative: Leifert & Steffan

(56) References cited:
- WO-A-2006/006654
- WO-A-2006/006664
- JP-A- 6 064 124
- JP-A- 2005 087 719
- US-A1- 2005 271 857

## Description

### BACKGROUND OF THE INVENTION

### 1. Field of the Invention

The present invention relates to an expandable heating sheet, a method of manufacturing the same, and a heating sheet bag for the same, and more particularly, to an expandable heating sheet which can be used while being fitted to a bendable portion such as an elbow or a knee and can be manufactured at low costs with efficiency, a method of manufacturing the same, and a heating sheet bag for the same.

### 2. Description of the Related Art

Hitherto, there have been used disposable body warmers in which a heating composition containing oxidizable metal, activated carbon, inorganic electrolyte, water, water-retaining agent, and the like mixed with each other, and for generating heat when being brought into contact with oxygen in air is received in an air permeable flat bag, and in order to prevent the heating composition from being brought into contact with outside air and to prevent the water from evaporating to diffuse to an outside until the heating composition is used, the air permeable flat bag is hermetically sealed in a non-air permeable outer bag. There are commercially available various body warmers, having heating properties set depending on portions to be applied such as on body, in pocket, and in a shoe. In order to facilitate attachment at a time of use, those body warmers are each provided with an adhesive layer.

There are also used disposable body warmers of a sheet type in which the heating composition is retained in spaces of a non-woven cloth, thereby preventing moving toward one side during the use.

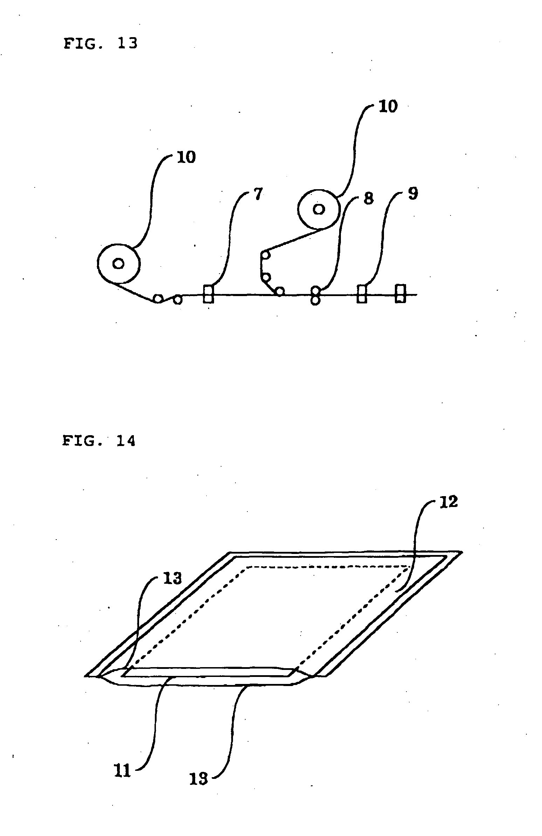

In a method of manufacturing a disposable body warmer of this type, for example, as shown in FIG. 13, onto a sheet-like non-woven cloth continuously supplied through rotating rolls, a heating composition and thermal fusion-bondable resin powder are sprayed, after that, another sheet-like non-woven cloth to be continuously supplied is overlapped thereon, the resultant is combined in a single body by heating and compression bonding by a compressor, the resultant is cut into a desired size, and the resultant is hermetically sealed in an outer bag.

Further, as disposable body warmers other than the above-mentioned body warmer, there are provided body warmers developed with an object of being fitted to a bendable portion such as an elbow or a knee to be used. There are listed, for example, as disclosed in Japanese Utility Model Application Laid-open No. Hei 06-26829, a disposable body warmer in which a heating portion filled with a heating agent (heating composition) is divided into a plurality of sections by a seal portion, and as disclosed in Japanese Utility Model Application Laid-open No. Hei 06-61222, an expandable disposable body warmer having a structure in which a disposable bodywarmer is provided to a desired surface of an expandable material, thereby fitting a body.

However, the portable bodywarmer, in which the heating portion charged with the heating composition is divided into the plurality of sections, can easily be bent at the seal portion even when the heating composition thereof is hardened due to reaction with oxygen, but is not expandable at the seal portion, so expansion property cannot be obtained for the body warmer as a whole. Further, with the article in which the disposable body warmer is provided to the desired surface of the expandable material, an attachment portion of the disposable body warmer is hardened as reaction of the heat composition with oxygen proceeds, so the disposable body warmer is hard and has no expansion property in the attachment portion.

WO 2006/006664 discloses a flexible heating element that can be secured to any part of the body by sticking or winding, being stretchable in accordance with the movement of the body to thereby be free from coming off or disengagement. There is provided a flexible heating element comprising a base material and a covering material and, interposed therebetween, multiple exothermic composition moldings, the exothermic composition moldings at peripheries thereof heat sealed to thereby create segmented heating parts and segmenting parts.

### SUMMARY OF THE INVENTION

It is therefore an object of the present invention to provide an expandable heating sheet, which does not lose its expansion property even when a heating composition thereof is hardened due to reaction with oxygen, can be used while being fitted to a bendable portion such as an elbow or a knee, and can be easily manufactured with efficiency, and a heating sheet bag in which the expandable heating sheet is hermetically sealed.

The inventors of the present invention have made a keen examination to solve the above-mentioned problems, thereby finding that, in a heating sheet obtained by overlapping laminate package materials each containing a thermal fusion-bondable seal material and a stretchable base to both surfaces of a plurality of flat heating bodies arranged an a plane such that thermal fusion-bondable surfaces of the laminate package materials are directed inwardly, and heating and compression bonding the resultant, to thereby combining the resultant in a single body by using, as each thermal fusion-bondable seal material, a thermal fusion-bondable seal material having a repetitive waveform, a thermal fusion-bondable seal material having a repetitive cancavo-convex form, or a thermal fusion-bondable seal material having a plurality of penetration portions, expansion property can easily be obtained. As a result, the inventors of the present invention attains an expendable heating sheet as claimed in claim 1, a method of manufacturing the same as claimed in claim 7, and a heating sheet bag for the same as claimed in claim 6.

That is, the present invention provides an expandable heating sheet according to claim 1 including a plurality of flat heating bodies arranged an a plane and laminate package materials each including a thermal fusion-bondable seal material having a repetitive waveform and a stretchable base, the laminate package materials being heated and compression bonded to both surfaces of the plurality of flat heating bodies.

Further, the present invention provides an expandable heating sheet according to claim 1 including a plurality of flat heating bodies arranged on a plane and laminate package materials each including a thermal fusion-bondable seal material having a repetitive concave-convex form and a stretchable base, the laminate package materials being heated and compression bonded to both surfaces of the plurality of flat heating bodies.

Further, the present invention provides an expandable heating sheet according to claim 1 including a plurality of flat heating bodies arranged on a plane and laminate package materials each including a thermal fusion-bondable seal material having a plurality of penetration portions and a stretchable base, the laminate package materials being heated and compression bonded to both surfaces of the plurality of flat heating bodies.

Further, the present invention provides an expandable heating sheet according to claim 1 including a plurality of flat heating bodies arranged on a plane and two laminate package materials which are heated and compression bonded to both surfaces of the plurality of flat heating bodies and are of one type or two types selected from the group consisting of: a laminate package material including a thermal fusion-bondable seal material having a repetitive waveform and a stretchable base; a laminate package material including a thermal fusion-bondable seal material having a repetitive concavo-convex form and the stretchable base; and a laminate package material including a thermal fusion bondable seal material having a plurality of penetration portions and the stretchable base.

Further, the present invention provides a heating sheet bag according to claim 6 including: the expendable heating sheet according to any one of the above-mentioned aspects; and a non-air permeable flat bag, in which the expandable heating sheet is hermetically sealed in the non-air permeable flat bag.

Further, the present invention provides a method of manufacturing an expendable heating sheet according to claim 7, including the steps of: arranging a plurality of flat heating bodies on a surface on a thermal fusion-bondable surface side of one laminate package material selected from the group consisting of: a laminate package material including a thermal fusion-bondable seal material having a repetitive waveform and a stretchable base; a laminate package material including a thermal fusion-bondable seal material having a repetitive concavo-convex form and the stretchable base; and a laminate package material including a thermal fusion-bondable seal material having a plurality of penetration portions and the stretchable base; wherein the thermal fusion-bondable seal material comprises a thermal fusion-bondable sheet, a rubber sheet, and a thermal fusion-bondable sheet stuck in the stated order; and wherein: temperatures of the respective thermal fusion-bondable sheets, at which thermal fusion-bondable components used for the thermal fusion-bondable sheets perform fusion-bonding, are different from each other; and one of the thermal fusion-bondable sheets, of which the temperature at which the thermal fusion-bondable component performs the fusion-bonding is lower than the temperature of another one of the thermal fusion-bondable sheets, and the stretchable base are stuck to each other; overlapping one laminate package material selected from the laminate package materials of three types on another-side surfaces of the plurality of flat heating bodies so that the thermal fusion-bondable surface of the one laminate package material is directed to a side of the plurality of flat heating bodies; and combining the one laminate package material, the plurality of flat heating bodies, and the one laminate package material into a single body by heating and compression bonding.

In the expandable heating sheet according to the present invention, a thermal fusion-bondable seal material having a stretchability or expansion property which is reduced by being heated is used as the thermal fusion-bondable seal material having the repetitive waveform, the thermal fusion-bondable seal material having the repetitive concavo-convex form, or the thermal fusion-bondable seal material having the plurality of penetration portion, so the expansion property is available in a waveform seal portion, a convex seal portion (portion having small thickness), or the penetration portion. Accordingly, the expandable heating sheet according to the present invention can be used while being fitted to the bendable portion such as an elbow or a knee. Further, in the method of manufacturing an expandable heating sheet according to the present invention, a direction in which the expandable heating sheet does not have the expansion property is set to a supply direction (direction in which tensile force is applied), or released paper or the like is laid under the expandable heating sheet to temporality eliminate the expansion property, thereby making it possible to easily manufacture the expandable heating sheet with efficiency by using a conventionally used manufacturing device without using a special manufacturing device.

### BRIEF DESCRIPTION OF THE DRAWINGS

In the accompanying drawings:
FIG. 1 is a sectional view showing an example of a structure of an expandable heating sheet before heating and compression bonding according to the present invention (first embodiment);
FIG. 2 is a sectional view showing an example of a structure of an expandable heating sheet before heating and compression bonding according to the present invention (second embodiment);
FIG. 3 is a sectional view showing an example of a structure of an expandable heating sheet before heating and compression bonding according to the present invention (third embodiment);
FIGS. 4 are sectional views each showing an example of a structure of the expandable heating sheet according to the present invention;
FIG. 5 is a perspective view showing an example of a laminate package material used for the expandable heating sheet according to the present invention (first embodiment, having expansion property in one direction);
FIG. 6 is a perspective view showing an example of a laminate package material used for the expandable heating sheet according to the present invention (second embodiment, having expansion property in one direction);
FIG. 7 is a perspective view showing an example of a laminate package material used for the expandable heating sheet according to the present invention (third embodiment, having expansion property in one direction);
FIG. 8 is a perspective view showing an example of a laminate package material used for the expandable heating sheet according to the present invention (first embodiment, having expansion property in two directions, that is, lengthwise direction and width direction);
FIG. 9 is a perspective view showing an example of a laminate package material used for the expandable heating sheet according to the present invention (second embodiment, having expansion property in two directions, that is, lengthwise direction and width direction);
FIG. 10 is a perspective view showing an example of a laminate package material used for the expandable heating sheet according to the present invention (third embodiment, having expansion property in two directions, that is, lengthwise direction and width direction);
FIG. 11 is a perspective view showing an example of a state where heating bodies are arranged on the laminate package material in the present invention;
FIG. 12 is a perspective view showing an example of a state where the laminate package materials are overlapped on both surfaces of the heating bodies such that thermal fusion-bondable surfaces of the laminate package materials are directed inwardly in the present invention;
FIG. 13 is a structural view showing an example of a manufacturing device for the expandable heating sheet according to the present invention; and
FIG. 14 is a perspective view showing an example of a heating sheet bag according to the present invention (perspective view with a part being eliminated).

### DETAILED DESCRIPTION OF THE PREFERRED EMBODIMENTS

An expandable heating sheet, a method of manufacturing the same, and a heating sheet bag are applied to a disposable body warmer for warming bendable portions such as elbows and knees, and a method of manufacturing the same.

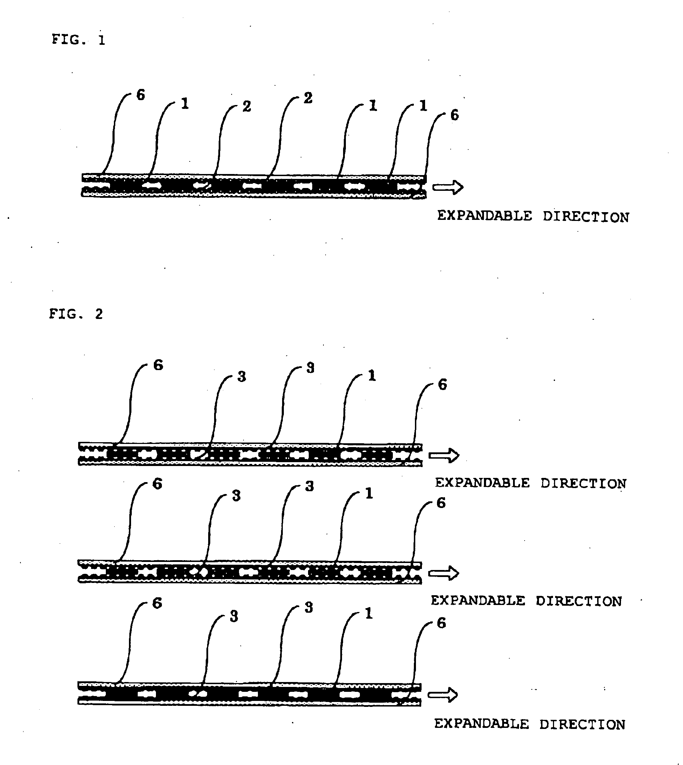

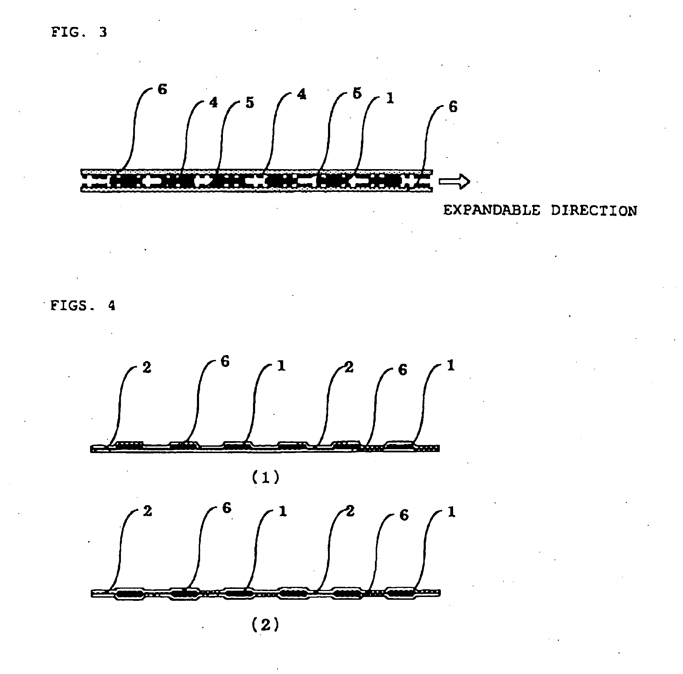

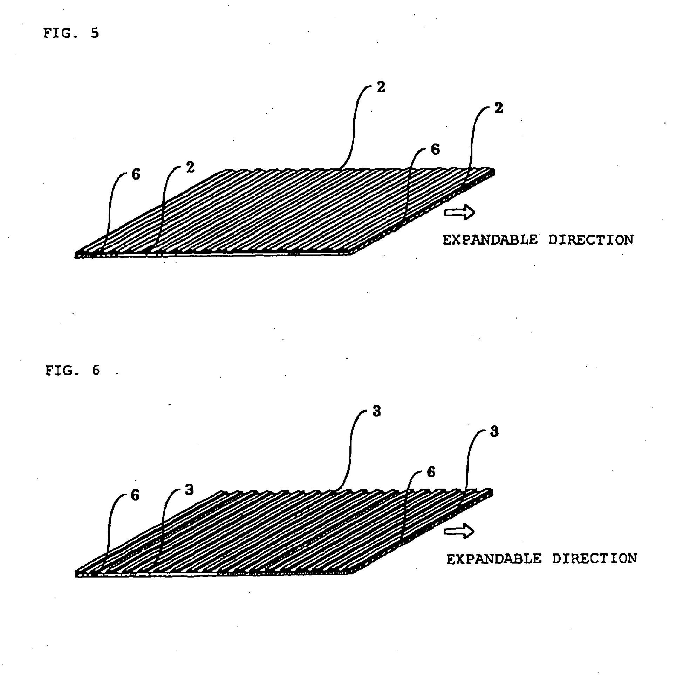

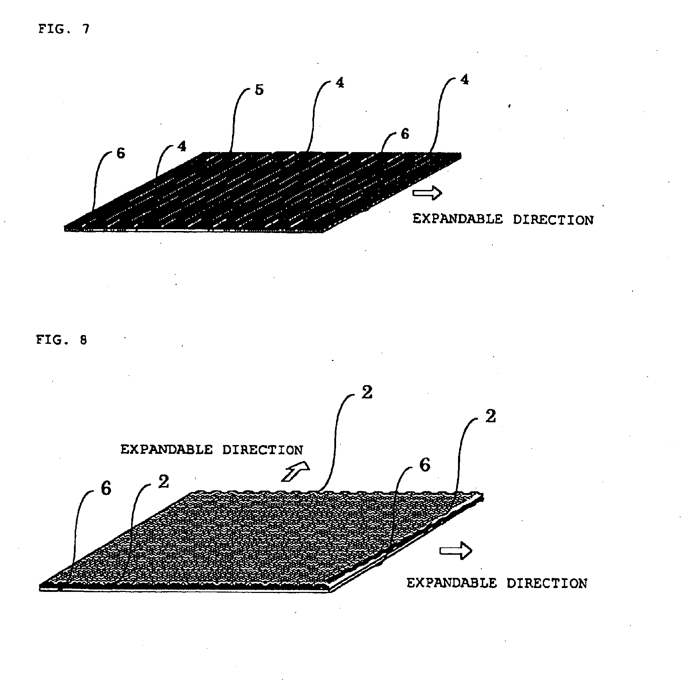

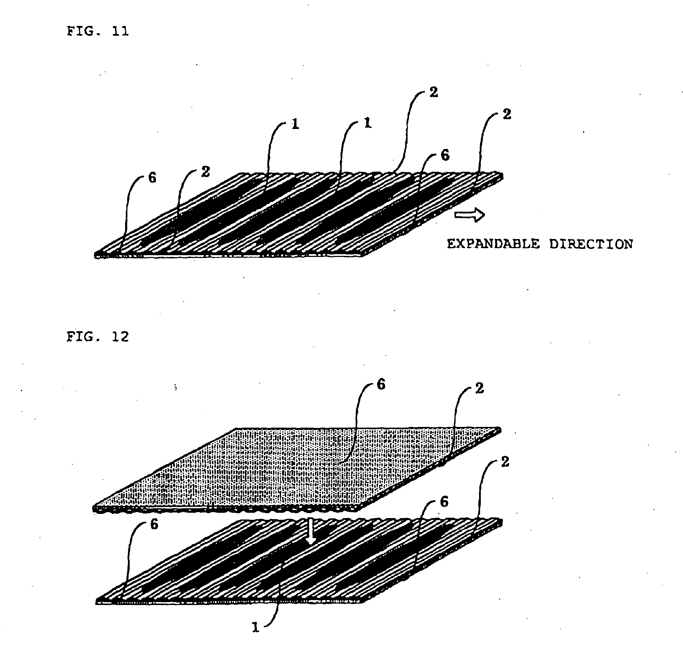

Hereinafter, detailed descriptions will be made of the expandable heating sheet according to claim 1 and its further embodiments according to claims 2 to 5, the method of manufacturing the same according to claim 7 and its embodiments according to claims 8 to 10, and the heating sheet bag according to claim 6 of the present invention with reference to FIGS. 1 to 14. However, the present invention is not limited by the description. Any non-claimed aspects are for illustrative purposes only: FIGS. 1 to 3 are sectional views each showing an example of a structure of the expandable heating sheet before, heating and compression bonding according to the present invention. FIGS. 4 are sectional views each showing an example of a structure of the expandable heating sheet according to the present invention. FIGS. 5 to 7 are perspective views each showing an example of a laminate package material (having expansion property in one direction) used for the expandable heating sheet according to the present invention. FIGS. 8 to 10 are perspective views each showing an example of a laminate package material (having expansion property in two directions, that is, lengthwise direction and width direction) used for the expandable heating sheet according to the present invention. FIG. 11 is a perspective view showing an example of a state where heating bodies are arranged on the laminate package material in the present invention. FIG. 12 is a perspective view showing an example of a state where the laminate package materials are overlapped on both surfaces of the heating bodies such that thermal fusion-bondable surfaces of the laminate package materials are directed inwardly in the present invention. FIG. 13 is a structural view showing an example of a manufacturing device for the expandable heating sheet according to the present invention. FIG. 14 is a perspective view (perspective view with a part being eliminated) showing an example of a heating sheet bag according to the present invention.

An expandable heating sheet according to a first embodiment of the present invention has a structure in which a plurality of flat heating bodies 1 arranged on a plane and two laminate package materials (for example, package material as shown in FIG. 5 or 8) each including a thermal fusion-bondable seal material 2 having a repetitive waveform and a stretchable base 6 are overlapped with each other such that the thermal fusion-bondable seal materials 2 are directed inwardly as shown in FIG. 1, to be heated and compression bonded to each other.

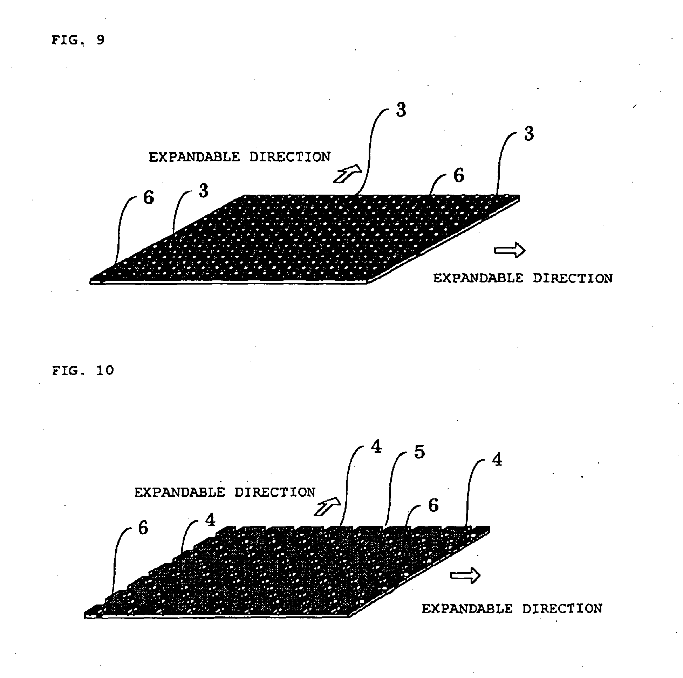

An expandable heating sheet according to a second embodiment of the present invention has a structure in which the plurality of flat heating bodies 1 arranged on a plane and two laminate package materials (for example, package material as shown in FIG. 6 or 9) each including a thermal fusion-bondable seal material 3 having a repetitive concavo-convex form and the stretchable base 6 are overlapped with each other such that the thermal fusion-bondable seal materials 3 are directed inwardly as shown in FIG. 2, to be heated and compression bonded to each other.

An expandable heating sheet according to a third embodiment of the present invention has a structure in which the plurality of flat heating bodies 1 arranged on a plane and two laminate package materials (for example, package material as shown in FIGS. 7 and 10) each including a thermal fusion-bondable seal material 5 having a plurality of penetration portions 4 and the stretchable base 6 are overlapped with each other such that the thermal fusion-bondable seal materials 5 are directed inwardly as shown in FIG. 3, to be heated and compression bonded to each other.

An expandable heating sheet according to a fourth embodiment of the present invention has a structure in which the laminate package materials heated and compression bonded to the both surfaces of the heating bodies 1 of the expandable heating sheet according to the first to third embodiments of the present invention are changed to laminate package materials of types different from each other.

Note that, for example, in a case of the expandable heating sheet according to the first embodiment of the present invention, the expandable heating sheet according to the present invention is finally formed to be ones shown in FIGS. 4. In the expandable heating sheet of each of FIGS. 4, portions of the thermal fusion-bondable seal materials 2 (exclusive of portions of heating bodies 1) having the repetitive waveform serves to impart expansion property to the expandable heating sheet. This also applies to a case of the expandable heating sheet according to the second to fourth embodiments.

The laminate package material used in the expandable heating sheet according to the present invention is a package material finished in each mode as shown in FIGS. 5 to 10 by overlapping the thermal fusion-bondable seal material including a thermal fusion-bondable component such as polyethylene or polypropylene, and the stretchable base each other and heating and fusion-bonding the thermal fusion-bondable seal material and the stretchable base to each other by using a roll, a mold, a compressor, or the like whose compression surface has a waveform or a concavo-convex form, or by overlapping the thermal fusion-bondable seal material including the plurality of penetration portions, and the stretchable base each other and heating and fusion-bonding the thermal fusion-bondable seal material and the stretchable base to each other by using a roll, a mold, a compressor, or the like whose compression surface is a flat surface. Further, in a case where, as the laminate package material, a material having expansion property in one direction (FIGS. 5 to 7) is used, when the laminate package materials are overlapped on the both surfaces of the heating bodies, the two laminate package materials are arranged such that expandable directions of those coincide with each other.

Note that the thermal fusion-bondable seal material used in the present invention include, in addition to the thermal fusion-bondable component such as polyethylene or polypropylene, a rubber component such as polybutadiene rubber, polyisoprene rubber, styrene-butadiene rubber, or polyurethane rubber, including as a monomer component, butadiene, isoprene, styrene, or urethane, respectively. Examples of the thermal fusion-bondable seal material include a thermal fusion-bondable seal material obtained by sticking a sheet containing the rubber component and a sheet containing the thermal fusion-bandable component to each other, a thermal fusion-bondable seal material including the sheet containing the rubber component and having a surface impregnated with thermal fusion-bondable resin powder, and a thermal fusion-bondable seal material obtained by mixing the rubber component with the thermal fusion-bondable component, and then melting and stretching the resultant.

Of those, according to the invention the thermal fusion-bondable seal material obtained by sticking the sheet containing the rubber component (hereinafter sometimes referred to as rubber sheet) to the sheet containing the thermal fusion-bondable component (hereinafter, sometimes referred to as thermal fusion-bondable sheet) is used, and the thermal fusion-bondable seal material has a three-layer structure including the thermal fusion-bondable sheet, the rubber sheet, and the thermal fusion-bondable sheet which are stuck to each other in the stated order. In this case, temperatures at which fusion-bonding is performed of the thermal fusion-bondable components used for the thermal fusion-bonding sheets are different from each other. It is according to the invention that any one of the thermal fusion-bondable sheets, containing the thermal fusion-bondable component whose fusion-bonding temperature is low, be stuck to the stretchable base.

Further, the stretchable base used in the present invention may be one which does not contract after stretching once or may be one which freely stretches and contracts, so there is no particular limitation. For example, various woven cloths and non-woven cloths may be used therefor. Of those, the non-woven cloth, which moderately stretches and has a nice texture is preferred.

In the present invention, the laminate package material according to the first embodiment of the present invention (FIGS. 5 and 8) stretches and contracts in the waveform portion of the thermal fusion-bondable seal material 2, the laminate package material according to the second embodiment of the present invention (FIGS. 6 and 9) stretches and contracts in concave portions (portions having small thickness) of the thermal fusion-bondable seal material 3, and the laminate packagematerial according to the third embodiment of the present invention (FIGS. 7 and 10) stretches and contracts in the portions of the penetration portions 4 of the thermal fusion-bondable seal material 5, thereby obtaining expansion property. A maximum stretching ratio ((L - L₀)/L₀) in a direction in which the laminate package material has expansion property is normally 30 to 200% at a room temperature (25°C). A maximum stretching ratio in a direction inwhich the laminate package material does not have expansion property is normally less than 30% at the room temperature (25°C). The expandable heating sheet according to the present invention uses the laminate package material. Therefore, the portions where the heating body is not provided serve to impart expansion property to the expandable heating sheet.

In the laminate package material according to the first embodiment of the present invention (FIGS. 5 and 8), an example of a sectional shape of the waveform of the thermal fusion-bondable seal material 2 includes a shape obtained by using a semicircular shape, a semielliptical shape, an arch shape, a mountain shape (trapezoidal shape), a U shape, a V shape, or a shape similar to those, one of those shapes being repetitively joined while being rotated by 180 degrees about an end portion of the shape as a center. Further, a height of the waveform (distance between highest portion and lowest portion) is normally 0.05 to 2 mm, and is preferably 0.1 to 1 mm. A width of the waveform (wavelength) is normally 0.5 to 20 mm, and is preferably 1 to 10 mm.

In the laminate package material according to the second embodiment of the present invention, a sectional shape of each convex portion of the thermal fusion-bondable seal material 3 as shown in FIG. 6 may be, for example, a semicircle, a semiellipse, a square, a rectangle, a trapezoid, a triangle, or a shape similar to those. A shape of the each convex portion of the thermal fusion-bondable seal material 3 as shown in FIG. 9 may be, for example, a circular columnar shape, a semispherical shape, a semielliptical shape, a cubic shape, a rectangular parallelepiped shape, a truncated cone shape, a truncated pyramid shape, a cone shape, a pyramid shape, or a shape similar to those. Further, a height of the convex portion with respect to the concave portion is normally 0.02 to 2 mm, and is preferably 0.05 to 1 mm. A width of the convex portion and the concave portion is normally 0,5 to 20 mm, and is preferably 1 to 10 mm. Note that, when the thermal fusion-bondable seal material is formed into a flat sheet with no convex portion and convex portion and a thickness of the thermal fusion-bondable seal material is made small, expansion property is obtained, but there are problems in that the thermal fusion-bondable seal material is easily ripped when being heated and compression bonded and an fusion-bonding strength with respect to the heating body is weakened, thereby causing the thermal fusion-bondable seal material to be easily removed. In the present invention, an apex of the each convex portion of the thermal fusion-bondable seal material is heated and compression bonded to the heating body, so there is no problem as described above.

In the laminate package material according to the third embodiment of the present invention, a planer shape of the penetration portion 4 having an oblong shape as shown in FIG. 7 may be, for example, an ellipse, a semicircle, a rectangle, a trapezoid, a triangle, or a shape similar to those. A planer shape of the penetration portion 4 as shown in FIG. 10 may be, for example, in addition to the above-mentioned shapes, a circular shape or a square shape. A width of the penetration portion of the oblong shape as shown in FIG. 7 is normally equal to or lower than 20 mm (including a width of a case where cut is simply provided), and is preferably 1 to 10 mm. Further, a size of the penetration portion as shown in FIG. 10 is normally 1 to 30 mm, and is preferably 3 to 15 mm in a case where the size is converted into that of the square shape. Note that, in the laminate package material according to the third embodiment of the present invention (FIGS. 7 and 10), it is preferred that the penetration portions (cutout portions) be also provided in a peripheral portion thereof such that the expansion property is imparted to an entire portion of the laminate package material.

In the expandable heating sheet and the method of manufacturing the same according to the present invention, as shown in FIG. 11, on a surface on the thermal fusion-bondable surface (thermal fusion-bondable seal material) side of the laminate package material, there are arranged the plurality of flat heating bodies 1. A shape of the heating body is not particularly limited. Examples of the shape may include a circular shape, a semielliptical shape, a semicircular shape, a square shape, a rectangular shape, a polygonal shape, a trapezoidal shape, a rhomboid shape, a parallelogram shape, a triangular shape, and a shape similar to those. However, the heating body does not have expansion property, so it is necessary to arrange the heating bodies so as not to inhibit the expansion property of the expandable heating sheet as much as possible. For example, the maximum stretching ratio ((L - L₀)/L₀) based on the expansion property in one direction tends to be substantially proportional to a value obtained by subtracting a length corresponding to portions of the heating bodies from a length corresponding to a portion having the expansion property.

In the present invention, for a structural material of the heating body, there are used a heating composition which generates heat by coming into contact with oxygen in air, for example, a material including oxidizable metal such as iron, activated carbon, inorganic electrolyte such as sodium chloride, water, and a water retention agent. However, it is necessary that the plurality of flat heating bodies be fixed to the surface of the laminate package material as shown in FIG. 11, the material preferably include a thickening agent in addition to the above-mentioned components such that the fixation can be performed on the surface of the laminate package material by application or printing. Normal ratios of the components are as follows. That is, a ratio of the oxidizable metal is 20 to 80 wt%, a ratio of the activated carbon is 1 to 10 wt%, a ratio of the inorganic electrolyte is 1 to 10 wt%, a ratio of water is 5 to 50 wt%, a ratio of water retention agent is 1 to 10 wt%, and a ratio of the thickening agent is 0.1 to 10 wt%.

In the expandable heating sheet and the method of manufacturing the same according to the present invention, on the surface of the laminate package material, the plurality of flat heating bodies are arranged and each are laid on one side thereof. After that, as shown in FIG. 12, on another-side surfaces of the heating bodies, the laminate package material is overlapped such that the thermal fusion-bondable surface thereof is directed to the heating body side, and the resultant is heated and compressionbonded to be combined into a single body. In this case, the two laminate package materials may be of different types, but are preferably of the same type. Further, in a case where a package material having expansion property in one direction is used, the two laminate package materials are arranged such that expandable directions of those coincide with each other.

Note that, in the present invention, at least one of the two laminate package materials may be an air permeable package material. Further, in order to use the expandable heating sheet while being fusion-bonded to a bendable portion such as an elbow or a knee, a glue layer may be provided on the surface on the opposite side of the thermal fusion-bondable surface of the one laminate package material, preferably, the laminate package material having no air permeability. The glue layer is covered with release paper or the like during a period prior to use.

The expandable heating sheet according to the present invention can be successively manufactured by setting a roll 10 around which the laminate package material is wound to a manufacturing device shown in FIG. 13 including a heating body arranging (applying/printing) portion 7, a heating and compression bonding portion 8, and a cutting portion 9, and starting up a manufacturing device. In this case, when a direction in which the laminate package material has the expansion property is set to a supply direction (direction in which tensile force is applied), due to the expansion property, it is difficult to maintain a constant supply rate. However, by setting a direction in which the laminate package material does not have the expansion property to the supply direction, the expandable heating sheet can be manufactured easily with efficiency. Further, in a case where the laminate package material has the expansion property in two directions, that is, the lengthwise direction and the width direction, release paper or the like is laid under the laminate package material to temporality eliminate the expansion property, thereby making it possible to easily manufacture the expandable heating sheet with efficiency.

The heating sheet bag according to the present invention includes, as shown in FIG. 14, an expandable heating sheet 11 as described above and a flat bag 12 with no air permeability, the expandable heating sheet 11 being hermetically sealed in the flat bag 12. In a case of manufacturing the heating sheet bag, for example, two non-air permeable package materials 13 made of polyethylene, polypropylene, or the like are overlapped with each other, and are heated and fusion-bonded to each other in a peripheral portion except one side to form a bag shape, after that, the expandable heating sheet 11 is inserted therein, and two non-air permeable package materials 13 are heated and fusion-bonded to each other on the remaining one side, thereby achieving the manufacture.

### Examples

Next, examples of the present invention will be described in detail, but the present invention is not limited by the examples.

### Example 1

### (Manufacture of Laminate Package Material)

As a thermal fusion-bondable seal material, a commercial thermal fusion-bondable sheet (thickness: 70µm) including a thermal fusion-bondable component sheet (low-temperature fusion-bonding), a rubber component sheet, and a thermal fusion-bondable component sheet (high-temperature fusion-bonding) was used. As a stretchable base, a commercial non-woven cloth (polyester spunlace) (thickness: 200 µm) was used. The thermal fusion-bondable seal material and the stretchable base were overlapped with each other such that a surface of a thermal fusion-bondable component (low-temperature fusion-bonding) of those is directed inwardly. Both surfaces thereof were heated and fusion-bonded to each other by being sandwiched by two molds each having a repetitive waveform in one direction. As a result, the laminate package material including the thermal fusion-bondable seal material 2 and the stretchable base 6 and having a repetitive waveform as shown in FIG. 5 was obtained. Note that, when the heating and compression bonding were performed, a temperature of the molds was set to a temperature at which the thermal fusion-bondable component sheet (low-temperature fusion-bonding) melts and the thermal fusion-bondable component sheet (high-temperature fusion-bonding) does not melt. A height of the waveform of the laminate package material (distance between highest portion and lowest portion) was 0.2 mm, a width of the waveform (wavelength) was 6 mm. Further, the laminate package material was cut into a size of 100 mm by 150 mm. The two laminate package materials structured as described above were manufactured and a surface of one of those was provided with pinholes thereby being air permeable.

### (Manufacture of Expandable Heating Sheet and Heating Sheet Bag)

Under a nitrogen gas atmosphere, 30 g of a coating liquid obtained by mixing 63.1 wt% of iron powder, 3.2 wt% of activated carbon, 1.9 wt% of salt (inorganic electrolyte), 26.5 wt% of water, 0.6 wt% of a water retention agent, and 4.7 wt% of a thickening agent was divided to be applied to six positions on a surface on a thermal fusion-bondable surface side of the laminate package material (non-air permeable), thereby obtaining the laminate package material on which heating bodies are arranged as shown in FIG. 11. Note that a shape of each heating body was an ellipse having a major-axis diameter of 80mm, a minor-axis diameter of 15 mm, and a thickness of 1.0 mm.

Next, as shown in FIG. 12, on surfaces of the heating bodies, the laminate package material (air permeable) was overlapped such that a thermal fusion-bondable surface of the laminate package material is directed to the heating body side and expandable directions of the laminate package materials coincide with each other, and the resultant was heated and compression bonded to each other to be combined in a single body, thereby obtaining a heating sheet as shown in FIG. 4A. Further, in order to use the expandable heating sheet while fusion-bonding the expandable heating sheet to a bendable portion such as an elbow or a knee, a surface of the non-air permeable laminate package material was provided with a glue layer and the glue layer was covered with release paper.

The expandable heating sheet was hermetically sealed in a non-air permeable flat bag having a size of 130 mm by 180 mm, thereby manufacturing a heating sheet bag.

### (Research on Expansion Property and Heating Property of Expandable Heating Sheet)

The two heating sheet bags described above were manufactured and were left for one day and night in a room at 25°C. The expandable heating sheet was taken out from one of the heating sheet bags. A maximum stretching ratio in a direction in which the expandable heating sheet has expansion property and a maximum stretching ratio in a direction in which the expandable heating sheet does not have the expansion property were immediately measured. As a result, the maximum stretching ratios were 50% and 3%, respectively.

Next, the expandable heating sheet was taken out from the other heating sheet bag, and was left on a cushion in a room at 25°C, and research was then performed on heating property thereof. As a result, a maximum temperature of the expandable heating sheet was 50°C, and a time in which a temperature of the expandable heating sheet was equal to or more than 40°C was six hours. Therefore, it was confirmed that the expandable heating sheet had favorable heating property.

Further, the expandable heating sheet after use (after temperature decrease) was measured for a maximum stretching ratio in the direction in which the expandable heating sheet has the expansion property and a maximum, stretching ratio in the direction in which the expandable heating sheet does not have the expansion property As a result, it was determined that the maximum stretching ratios were 48% and 2% which were substantially the same as those in the case before use.

### Example 2

### (Manufacture of Expandable Heating Sheet and Heating Sheet Bag)

In this example, except that, in the manufacture of the laminate package material according to Example 1, in place of the two molds each having the repetitive waveform in one direction, a mold having a repetitive concavo-convex form in one direction and a flat mold were used, in the same manner as that of Example 1, the laminate package material including the thermal fusion-bondable seal material 3 having the repetitive concavo-convex form and the stretchable base 6 as shown in FIG. 6 was manufactured. Note that a thickness of the convex portion of the laminate package material was 0.25 mm, a thickness of the concave portion thereof was 0.15 mm, and a width of each of the convex portion and the concave portion was 3 mm. Further, except that the laminate package material structured in this manner was used, the expandable heating sheet was manufactured in the same manner as that of Example 1. The expandable heating sheet was hermetically sealed in the non-air permeable flat bag, thereby obtaining a heating sheet bag.

### (Research on Expansion Property and Heating Property of Expandable Heating Sheet)

The two heating sheet bags described above were manufactured and were left for one day and night in a room at 25°C. The expandable heating sheet was taken out from one of the heating sheet bags. A maximum stretching ratio in a direction in which the expandable heating sheet has expansion property and a maximum stretching ratio in a direction in which the expandable heating sheet does not have the expansion property were immediately measured. As a result, the maximum stretching ratios were 40% and 3%, respectively.

Next, the expandable heating sheet was taken out from the other heating sheet bag, and was left on a cushion in a room at 25°C, and research was then performed on heating property thereof. As a result, a maximum temperature of the expandable heating sheet was 48°C, and a time in which a temperature of the expandable heating sheet was equal to or more than 40°C was seven hours. Therefore, it was confirmed that the expandable heating sheet had favorable heating property.

Further, the expandable heating sheet after use (after temperature decrease) was measured for a maximum stretching ratio in the direction in which the expandable heating sheet has the expansion property and a maximum stretching ratio in the direction in which the expandable heating sheet does not have the expansion property. As a result, it was determined that the maximum stretching ratios were 38% and 2% which are substantially the same as those in the case before use.

### Example 3

### (Manufacture of Expandable Heating Sheet and Heating Sheet Bag)

In this example, except that, in the manufacture of the laminate package material according to Example 1, a thermal fusion-bondable sheet was provided with slits each having a width of 1.0 mm at intervals of 5 mm, and that in place of the two molds each having the repetitive waveform, two flat molds were used, in the same manner as that of Example 1, the laminate package material including the thermal fusion-bondable seal material 5 having the plurality of oblong penetration portions 4 and the stretchable base 6 as shown in FIG. 7 was manufactured. Further, except that the laminate package material structured in this manner was used, the expandable heating sheet was manufactured in the same manner as that of Example 1. The expandable heating sheet was hermetically sealed in the non-air permeable flat bag, thereby obtaining a heating sheet bag.

### (Research on Expansion Property and Heating Property of Expandable Heating Sheet)

The two heating sheet bags described above were manufactured and were left for one day and night in a room at 25°C. The expandable heating sheet was taken out from one of the heating sheet bags. A maximum stretching ratio in a direction in which the expandable heating sheet has expansion property and a maximum stretching ratio in a direction in which the expandable heating sheet does not have the expansion property were immediately measured. As a result, the maximum stretching ratios were 42% and 2%, respectively.

Next, the expandable heating sheet was taken out from the other heating sheet bag, and was left on a cushion in a room at 25°C, and research was then performed on heating property thereof. As a result, a maximum temperature of the expandable heating sheet was 52°C, and a time in which a temperature of the expandable heating sheet was equal to or more than 40°C was five hours. Therefore, it was confirmed that the expandable heating sheet had favorable heating property.

Further, the expandable heating sheet after use (after temperature decrease) was measured for a maximum stretching ratio in the direction in which the expandable heating sheet has the expansion property and a maximum stretching ratio in the direction in which the expandable heating sheet does not have the expansion property. As a result, it was determined that the maximum stretching ratios were 40% and 1% which were substantially the same as those in the case before use.

### Example 4

### (Manufacture of Expandable Heating Sheet and Heating Sheet Bag)

In the same manner as that of Example 1, the laminate package material including the thermal fusion-bondable seal material 2 having the repetitive waveform and the stretchable base 6 as shown in FIG. 5 was obtained. Further, in the same manner as that of Example 2, the laminate package material including the thermal fusion-bondable seal material 3 having the repetitive concavo-convex form and the stretchable material 6 as shown in FIG. 6 was obtained, and the laminate package material was provided with pinholes formed in a surface thereof, thereby being air permeable. Except that those two types of laminate package materials were used, an expandable heating sheet was manufactured as in the same manner as that of Example 1. The expandable heating sheet was hermetically sealed in the non-air permeable flat bag, thereby obtaining a heating sheet bag.

### (Research on Expansion Property and Heating Property of Expandable Heating Sheet)

The two heating sheet bags described above were manufactured and were left for one day and night in a room at 25°C. The expandable heating sheet was taken out from one of the heating sheet bags. A maximum stretching ratio in a direction in which the expandable heating sheet has expansion property and a maximum stretching ratio in a direction in which the expandable heating sheet does not have the expansion property were immediately measured. As a result, the maximum stretching ratios were 50% and 2%, respectively.

Next, the expandable heating sheet was taken out from the other heating sheet bag, and was left on a cushion in a room at 25°C, and research was then performed on heating property thereof. As a result, a maximum temperature of the expandable heating sheet was 50°C, and a time in which a temperature of the expandable heating sheet was equal to or more than 40°C was eight hours. Therefore, it was confirmed that the expandable heating sheet had favorable heating property.

Further, the expandable heating sheet after use (after temperature decrease) was measured for a maximum stretching ratio in the direction in which the expandable heating sheet has the expansion property and a maximum stretching ratio in the direction in which the expandable heating sheet does not have the expansion property. As a result, it was determined that the maximum stretching ratios were 49% and 1% which were substantially the same as those in the case before use.

### Example 5

### (Manufacture of Expandable Heating Sheet and Heating Sheet Bag)

In this example, except that, in the manufacture of the laminate package material according to Example 1, in place of the two molds each having the repetitive waveform in one direction, two molds each having repetitive waveforms in two directions were used, in the same manner as that of Example 1, a laminate package material including the thermal fusion-bondable seal material 2 having the repetitive waveforms and the stretchable base 6 as shown in FIG. 8 was manufactured. Note that a height and a width of the waveform of the laminate package material were the same as those of Example 1. Further, except that the laminate package material was used and six heating bodies each having a diameter of 30 mm and a thickness of 1. 0 mmwere arranged, the expandable heating sheet was manufactured in the same manner as that of Example 1. The expandable heating sheet was hermetically sealed in the non-air permeable flat bag, thereby obtaining the heating sheet bag.

### (Research on Expansion Property and Heating Property of Expandable Heating Sheet)

The two heating sheet bags described above were manufactured and were left for one day and night in a room at 25°C. The expandable heating sheet was taken out from one of the heating sheet bags. Maximum stretching ratios in two directions in which the expandable heating sheet had expansion property, that is, the lengthwise direction and the width direction were immediately measured. As a result, the maximum stretching ratios were 30% and 24%, respectively.

Next, the expandable heating sheet was taken out from the other heating sheet bag, and was left on a cushion in a room at 25°C, and research was then performed on heating property thereof. As a result, a maximum temperature of the expandable heating sheet was 50°C, and a time in which a temperature of the expandable heating sheet was equal to or more than 40°C was six hours. Therefore, it was confirmed that the expandable heating sheet had favorable heating property.

Further, the expandable heating sheet after use (after temperature decrease) was measured for maximum stretching ratios in the two directions in which the expandable heating sheet has the expansion property, that is, the lengthwise direction and the width direction. As a result, it was determined that the maximum stretching ratios were 27% and 21% which were substantially the same as those in the case before use.

### Comparative Example 1

### (Manufacture of Expandable Heating Sheet and Heating Sheet Bag)

In this example, except that, in the manufacture of the laminate package material according to Example 1, in place of the two molds each having the repetitive waveform, two flat molds were used, in the same manner as that of Example 1, the laminate package material including a flat thermal fusion-bondable seal material and the stretchable base was manufactured. Further, except that the laminate package material structured in this manner was used, the expandable heating sheet was manufactured in the same manner as that of Example 1. The expandable heating sheet was hermetically sealed in the non-air permeable flat bag, thereby obtaining a heating sheet bag.

### (Research on Expansion Property and Heating Property of Expandable Heating Sheet)

The two heating sheet bags described above were manufactured and were left for one day and night in a room at 25°C. The expandable heating sheet was taken out from one of the heating sheet bags. Maximum stretching ratios in two directions, that is, the lengthwise direction and the width direction, were immediately measured. As a result, the maximum stretching ratios were 8% and 2%, respectively.

Next, the expandable heating sheet was taken out from the other heating sheet bag, and was left on a cushion in a room at 25°C, and research was then performed on heating property thereof. As a result, a maximum temperature of the expandable heating sheet was 50°C, and a time in which a temperature of the expandable heating sheet was equal to or more than 40°C was six hours. Therefore, it was confirmed that the expandable heating sheet had favorable heating property.

Further, the expandable heating sheet after use (after temperature decrease) was measured for maximum stretching ratios in the two directions, that is, the lengthwise direction and the width direction. As a result, it was determined that the maximum stretching ratios were 4% and 1% which were lower than the maximum stretching ratios before use.

As described above, the expandable heating sheet according to each of the examples of the present invention includes, as the thermal fusion-bondable seal material, the thermal fusion-bondable seal material having the repetitive waveform, the thermal fusion-bondable seal material having the repetitive concavo-convex form, or the thermal fusion-bondable seal material having the plurality of oblong penetration portions. Accordingly, the expansion property is provided to waveform seal portions, concave seal portions (portions having small thickness), or the penetration portions, so it is revealed that the expansion property can be maintained during or even after use.

## Claims

1. An expandable heating sheet, comprising:
a plurality of heating bodies (1) which are flat and arranged on a plane; and
two laminate package materials sandwiching the plurality of heating bodies from both sides thereof,
**characterized in that** the two laminate package materials are selected from the group consisting of: a laminate package material comprising a thermal fusion-bondable seal material (2) having a repetitive waveform and a stretchable base (6); a laminate package material comprising a thermal fusion-bondable seal material (3) having a repetitive concavo-convex form and the stretchable base (6); and a laminate package material comprising a thermal fusion-bondable seal material (5) having a plurality of penetration portions and the stretchable base (6);
wherein each thermal fusion-bondable seal material comprises a thermal fusion-bondable sheet, a rubber sheet, and a thermal fusion-bondable sheet stuck in the stated order; and
wherein: temperatures of the respective thermal fusion-bondable sheets, at which thermal fusion-bondable components used for the thermal fusion-bondable sheets perform fusion-bonding, are different from each other; and
the thermal fusion-bondable sheet, of which the temperature at which the thermal fusion-bondable component performs the fusion-bonding is lower , and the stretchable base are stuck to each other.

2. An expandable heating sheet, according to claim 1, wherein the stretchable base comprises a non-woven cloth.

3. An expandable heating sheet according to claim 1, wherein each of the plurality of heating bodies comprises oxidizable metal, activated carbon, inorganic electrolyte, water, a thickening agent, and polymeric water retention agent.

4. An expandable heating sheet according to claim 1, wherein at least one of the two laminate package materials comprises an air permeable package material.

5. An expandable heating sheet according to claim 1, further comprising a glue layer provided to one of the two laminate package materials on a surface opposite to a thermal fusion-bondable surface thereof.

6. A heating sheet bag, comprising:
the expandable heating sheet according to claim 1; and
a non-air permeable flat bag,
wherein the expandable heating sheet is hermetically sealed in the non-air permeable flat bag.

7. A method of manufacturing an expandable heating sheet of claim 1, comprising the steps of:
arranging a plurality of heating bodies which are flat on a surface on a thermal fusion-bondable surface side of one laminate package material selected from the group consisting of: a laminate package material comprising a thermal fusion-bondable seal material having a repetitive waveform and a stretchable base; a laminate package material comprising a thermal fusion-bondable seal material having a repetitive concavo-convex form and the stretchable base; and a laminate package material comprising a thermal fusion-bondable seal material having a plurality of penetration portions and the stretchable base;
wherein the thermal fusion-bondable seal material comprises a thermal fusion-bondable sheet, a rubber sheet, and a thermal fusion-bondable sheet stuck In the stated order; and
wherein: temperatures of the respective thermal fusion-bondable sheets, at which thermal fusion-bondable components used for the thermal fusion-bondable sheets perform fusion-bonding, are different from each other; and
one of the thermal fusion-bondable sheets, of which the temperature at which the thermal fusion-bondable component performs the fusion-bonding is lower than the temperature of another one of the thermal fusion-bondable sheets, and the stretchable base are stuck to each other;
overlapping one laminate package material selected from the laminate packages materials of three types on another-side surfaces of the plurality of flat heating bodies so that the thermal fusion-bondable surface of the one laminate package material is directed to a side of the plurality of heating bodies; and
combining the one laminate package material, the plurality of flat heating bodies, and the one laminate package material into a single body by heating and compression bonding.

8. A method of manufacturing an expandable heating sheet according to claim 7,
wherein the step of arranging the plurality of heating bodies comprises fixing a coating liquid including a heating composition, which generates heat by coming into contact with oxygen in air, to the surface on the thermal fusion-bondable surface side of one of the laminate package materials by one of application and printing.

9. A method of manufacturing an expandable heating sheet according to claim 7,
wherein the step of arranging the plurality of flat heating bodies comprises arranging the plurality of flat heating bodies so that each of the plurality of flat heating bodies has an oblong shape extending in a non-expandable direction.

10. A method of manufacturing an expandable heating sheet according to claim 7, further comprising the step of providing a glue layer on a surface opposite to the thermal fusion-bondable surface of one of the laminate package materials.

## Patentansprüche

1. Einmal-Heizdecke, umfassend:
eine Vielzahl von Heizelementen (1), die flach und in einer Ebene angeordnet sind; und
zwei Laminat-Packmaterialien, welche die Vielzahl von Heizelementen auf deren beiden Seiten umgeben,
**dadurch gekennzeichnet, dass** die beiden Laminat-Packmaterialien ausgewählt sind aus der Gruppe bestehend aus: einem Laminat-Packmaterial, das ein thermisch verschmelzbares Dichtmaterial (2) mit einer repetitiven Wellenform und einem dehnbaren Träger (6) umfasst; einem Laminat-Packmaterial, das ein thermisch verschmelzbares Dichtmaterial (3) mit einer repetitiven konkav-konvexen Form und dem dehnbaren Träger (6) umfasst; sowie einem Laminat-Packmaterial, das ein thermisch verschmelzbares Dichtmaterial (5) mit einer Vielzahl von Durchbruchsabschnitten (5) und dem dehnbaren Träger (6) umfasst;
wobei jedes thermisch verschmelzbare Dichtmaterial eine thermisch verschmelzbare Lage, eine Gummilage und eine thermisch verschmelzbare Lage umfasst, die in der angegebenen Reihenfolge aneinander haften; und
wobei die Temperaturen für die jeweiligen thermisch verschmelzbaren Lagen, bei denen die thermisch verschmelzbaren Komponenten, die für die thermisch verschmelzbaren Lagen verwendet werden, die thermische Verschmelzung bewirken, sich von einander unterscheiden; und
wobei die thermisch verschmelzbare Lage, für welche die Temperatur, bei der die thermisch verschmelzbare Komponente die Verschmelzung bewirkt, niedriger ist, und der dehnbare Träger aneinander haften.

2. Einmal-Heizdecke nach Anspruch 1, wobei der dehnbare Träger einen nicht verwebten Stoff umfasst.

3. Einmal-Heizdecke nach Anspruch 1, wobei jedes der Vielzahl von Heizelementen oxidierbares Metall, Aktivkohle, anorganisches Elektrolyt, Wasser, ein Verdickungsmittel und ein polymeres Wasserrückhaltemittel umfasst.

4. Einmal-Heizdecke nach Anspruch 1, wobei zumindest eines der beiden Laminat-Packmaterialien ein luftdurchlässiges Packmaterial umfasst.

5. Einmal-Heizdecke nach Anspruch 1, des Weiteren umfassend eine Klebstoffschicht, die auf einer der beiden Laminat-Packmaterialien auf einer der thermisch verschmelzbaren Oberfläche derselben gegenüberliegenden Oberfläche vorgesehen ist.

6. Heizdeckentasche, umfassend:
die Einmal-Heizdecke nach Anspruch 1; und
eine nicht luftdurchlässige flache Tasche,
wobei die Einmal-Heizdecke in der nicht luftdurchlässigen flachen Tasche hermetisch versiegelt ist.

7. Verfahren zur Herstellung einer Einmal-Heizdecke nach Anspruch 1, umfassend die Schritte:
Anordnen einer Vielzahl von Heizelementen, die flach sind, auf einer Oberfläche auf einer thermisch verschmelzbaren Oberflächenseite eines Laminat-Packmaterials, das ausgewählt ist aus der Gruppe bestehend aus: einem Laminat-Packmaterial, das ein thermisch verschmelzbares Dichtmaterial mit einer repetitiven Wellenform und einem dehnbaren Träger umfasst; einem Laminat-Packmaterial, das ein thermisch verschmelzbares Dichtmaterial mit einer repetitiven konkav-konvexen Form und dem dehnbaren Träger umfasst; sowie einem Laminat-Packmaterial, das ein thermisch verschmelzbares Dichtmaterial mit einer Vielzahl von Durchbruchsabschnitten und dem dehnbaren Träger umfasst;
wobei das thermisch verschmelzbare Dichtmaterial eine thermisch verschmelzbare Lage, eine Gummilage und eine thermisch verschmelzbare Lage umfasst, die in der angegebenen Reihenfolge aneinander haften; und
wobei die Temperaturen für die jeweiligen thermisch verschmelzbaren Lagen, bei denen die thermisch verschmelzbaren Komponenten, die für die thermisch verschmelzbaren Lagen verwendet werden, die thermische Verschmelzung bewirken, sich von einander unterscheiden; und
wobei eine der thermisch verschmelzbaren Lagen, für welche die Temperatur, bei der die thermisch verschmelzbare Komponente die Verschmelzung bewirkt, niedriger ist als die Temperatur einer weiteren der thermisch verschmelzbaren Lagen, und der dehnbare Träger aneinander haften;
Überlappen eines Laminat-Packmaterials, das aus den Laminat-Packmaterialien der drei Typen ausgewählt ist, auf anderen Seitenflächen der Vielzahl von flachen Heizelementen, so dass die thermisch verschmelzbare Oberfläche des einen Laminat-Packmaterials auf eine Seite der Vielzahl von Heizelementen gerichtet ist; und
Zusammenfügen des Laminat-Packmaterials, der Vielzahl von flachen Heizelementen und des einen Laminat-Packmaterials zu einem einzigen Körper durch Erhitzen und Kompressionsbonden.

8. Verfahren zur Herstellung einer Einmal-Heizdecke nach Anspruch 7, wobei der Schritt des Anordnens der Vielzahl von Heizelementen das Anbringen einer Beschichtungsflüssigkeit einschließlich einer Heizzusammensetzung, welche bei Kontakt mit Sauerstoff in der Luft Wärme erzeugt, auf die Oberfläche auf der thermisch verschmelzbaren Oberflächenseite auf einem der Laminat-Packmaterialien entweder durch Auftragen oder Aufdrucken umfasst.

9. Verfahren zur Herstellung einer Einmal-Heizdecke nach Anspruch 7, wobei der Schritt des Anordnens der Vielzahl von flachen Heizelementen das Anordnen der Vielzahl von flachen Heizelementen auf eine Weise umfasst, dass jedes der Vielzahl von flachen Heizelementen eine längliche Form aufweist, die sich in eine nicht erweiterbare Richtung erstreckt.

10. Verfahren zur Herstellung einer Einmal-Heizdecke nach Anspruch 7, des Weiteren umfassend den Schritt des Vorsehens einer Klebstoffschicht auf einer der thermisch verschmelzbaren Oberfläche eines der Laminat-Packmaterialien gegenüberliegenden Oberfläche.

## Revendications

1. Feuille chauffante extensible, comprenant :
une pluralité de corps chauffants (1) qui sont plats et disposés sur un plan ; et
deux matériaux d'emballage stratifiés prenant en sandwich la pluralité de corps chauffants de part et d'autre de ceux-ci,
**caractérisée en ce que** les deux matériaux d'emballage stratifiés sont sélectionnés dans le groupe composé de : un matériau d'emballage stratifié comprenant un matériau d'étanchéité liable par fusion thermique (2) ayant une forme d'onde répétitive et une base étirable (6) ; un matériau d'emballage stratifié comprenant un matériau d'étanchéité liable par fusion thermique (3) ayant une forme concave-convexe répétitive et la base étirable (6) ; et un matériau d'emballage stratifié comprenant un matériau d'étanchéité liable par fusion thermique (5) ayant une pluralité de parties de pénétration et la base étirable (6),
dans laquelle chaque matériau d'étanchéité liable par fusion thermique comprend une feuille liable par fusion thermique, une feuille de caoutchouc et une feuille liable par fusion thermique, collées dans cet ordre ; et
dans laquelle les températures des feuilles liables par fusion thermique respectives, auxquelles les composants liables par fusion thermique utilisés pour les feuilles liables par fusion thermique accomplissent la liaison par fusion, sont différentes l'une de l'autre ; et
la feuille liable par fusion thermique, dont la température à laquelle le composant liable par fusion thermique accomplit la liaison par fusion est plus faible, et la base étirable sont collées l'une avec l'autre.

2. Feuille chauffante extensible selon la revendication 1, dans laquelle la base étirable comprend un tissu non tissé.

3. Feuille chauffante extensible selon la revendication 1, dans laquelle chacun de la pluralité de corps chauffants comprend un métal oxydable, un charbon actif, un électrolyte inorganique, de l'eau, un épaississant et un agent de rétention d'eau polymère.

4. Feuille chauffante extensible selon la revendication 1, dans laquelle au moins un des deux matériaux d'emballage stratifiés comprend un matériau d'emballage perméable à l'air.

5. Feuille chauffante extensible selon la revendication 1, comprenant en outre une couche de colle fournie sur l'un des deux matériaux d'emballage stratifiés, sur une surface opposée à une surface liable par fusion thermique de celui-ci.

6. Poche de feuille chauffante, comprenant :
la feuille chauffante extensible selon la revendication 1 ; et
une poche plate non perméable à l'air,
dans laquelle la feuille chauffante extensible est enfermée hermétiquement dans la poche plate non perméable à l'air.

7. Procédé de fabrication d'une feuille chauffante extensible selon la revendication 1, comprenant les étapes consistant à :
disposer une pluralité de corps chauffants qui sont plats sur une surface sur un côté de surface liable par fusion thermique d'un matériau d'emballage stratifié sélectionné dans le groupe composé de : un matériau d'emballage stratifié comprenant un matériau d'étanchéité liable par fusion thermique ayant une forme d'onde répétitive et une base étirable ; un matériau d'emballage stratifié comprenant un matériau d'étanchéité liable par fusion thermique ayant une forme concave-convexe répétitive et la base étirable ; et un matériau d'emballage stratifié comprenant un matériau d'étanchéité liable par fusion thermique ayant une pluralité de parties de pénétration et la base étirable ;
dans lequel le matériau d'étanchéité liable par fusion thermique comprend une feuille liable par fusion thermique, une feuille de caoutchouc et une feuille liable par fusion thermique, collées dans cet ordre ; et
dans lequel les températures des feuilles liables par fusion thermique respectives, auxquelles les composants liables par fusion thermique utilisés pour les feuilles liables par fusion thermique accomplissent la liaison par fusion, sont différentes l'une de l'autre ; et
l'une des feuilles liables par fusion thermique, dont la température à laquelle le composant liable par fusion thermique accomplit la liaison par fusion est inférieure à la température d'une autre des feuilles liables par fusion thermique, et la base étirable sont collées l'une avec l'autre ;
déposer un matériau d'emballage stratifié sélectionné parmi les matériaux d'emballage stratifiés des trois types sur une surface d'un autre côté de la pluralité de corps chauffants plats de façon à ce que la surface liable par fusion thermique du matériau d'emballage stratifié soit tournée vers un côté de la pluralité de corps chauffants ; et
combiner le matériau d'emballage stratifié, la pluralité de corps chauffants plats et le matériau d'emballage stratifié en un seul corps par chauffage et liaison par compression.

8. Procédé de fabrication d'une feuille chauffante extensible selon la revendication 7, dans lequel l'étape de disposition de la pluralité de corps chauffants comprend la fixation d'un liquide de couchage comprenant une composition chauffante, qui génère de la chaleur en entrant en contact avec l'oxygène dans l'air, sur la surface sur le côté de surface liable par fusion thermique de l'un des matériaux d'emballage stratifiés par l'une ou l'autre d'une application et d'une impression.

9. Procédé de fabrication d'une feuille chauffante extensible selon la revendication 7, dans lequel l'étape de disposition de la pluralité de corps chauffants plats comprend la disposition de la pluralité de corps chauffants plats de façon à ce que chacun de la pluralité de corps chauffants plats ait une forme oblongue s'étendant dans une direction non extensible.

10. Procédé de fabrication d'une feuille chauffante extensible selon la revendication 7, comprenant en outre l'étape de fourniture d'une couche de colle sur une surface opposée à la surface liable par fusion thermique de l'un des matériaux d'emballage stratifiés.
